(19) Europäisches Patentamt
European Patent Office
Office européen des brevets

(11) **EP 2 884 886 B1**

(12) **EUROPEAN PATENT SPECIFICATION**

(45) Date of publication and mention
of the grant of the patent:
**25.03.2020 Bulletin 2020/13**

(51) Int Cl.:
*A61B 5/00* *(2006.01)*  *G01N 29/24* *(2006.01)*
*G01N 21/17* *(2006.01)*

(21) Application number: **13783991.6**

(22) Date of filing: **14.08.2013**

(86) International application number:
**PCT/IB2013/056632**

(87) International publication number:
**WO 2014/027316 (20.02.2014 Gazette 2014/08)**

(54) **COMPACT LASER AND EFFICIENT PULSE DELIVERY FOR PHOTOACOUSTIC IMAGING**

KOMPAKTLASER UND EFFIZIENTE PULSABGABE ZUR FOTOAKUSTISCHEN BILDGEBUNG

DISTRIBUTION D'IMPULSION EFFICACE ET LASER COMPACT ET POUR IMAGERIE PHOTOACOUSTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.08.2012 US 201261682817 P**

(43) Date of publication of application:
**24.06.2015 Bulletin 2015/26**

(73) Proprietors:
• **Koninklijke Philips N.V.**
**5656 AG Eindhoven (NL)**
• **Washington University**
**Saint Louis, MO 63130 (US)**

(72) Inventors:
• **MASLOV, Konstantin**
**5656 AE Eindhoven (NL)**
• **ERPELDING, Todd Nicholas**
**5656 AE Eindhoven (NL)**
• **JANKOVIC, Ladislav**
**5656 AE Eindhoven (NL)**
• **WANG, Lihong**
**5656 AE Eindhoven (NL)**

(74) Representative: **Philips Intellectual Property & Standards**
**High Tech Campus 5**
**5656 AE Eindhoven (NL)**

(56) References cited:
EP-A1- 0 352 923    WO-A1-2010/048258
WO-A1-2012/077356    WO-A2-2008/039988

• CHULHONG KIM ET AL: "Handheld array-based photoacoustic probe for guiding needle biopsy of sentinel lymph nodes", JOURNAL OF BIOMEDICAL OPTICS, vol. 15, no. 4, 1 January 2010 (2010-01-01), page 046010, XP055065232, ISSN: 1083-3668, DOI: 10.1117/1.3469829
• Lihong V. Wang ET AL: "Optimal beam size for light delivery to absorption-enhanced tumors buried in biological tissues and effect of multiple-beam delivery: a Monte Carlo study", Applied Optics, vol. 36, no. 31, 1 November 1997 (1997-11-01), page 8286, XP055578400, WASHINGTON, DC; US ISSN: 0003-6935, DOI: 10.1364/AO.36.008286
• FAVAZZA CHRISTOPHER P ET AL: "Optimal oblique light illumination for photoacoustic microscopy beyond the diffusion limit", PHOTONS PLUS ULTRASOUND: IMAGING AND SENSING 2011, SPIE, 1000 20TH ST. BELLINGHAM WA 98225-6705 USA, vol. 7899, no. 1, 10 February 2011 (2011-02-10), pages 1-6, XP060007514, DOI: 10.1117/12.874903 [retrieved on 2011-02-28]

**Description**

FIELD OF THE INVENTION

The present invention relates to photoacoustic (PA) probes and, more particularly, to PA probes for directing output from a wavelength conversion assembly.

BACKGROUND OF THE INVENTION

A sentinel lymph node biopsy is a means by which to stage cancer and to judge the risk that it will metastasize via the lymphatic system. The sentinel lymph node is the first node to receive lymphatic drainage from the tumor. If cancer has spread to the sentinel lymph node, it may have spread further. A radioactive tracer and/or a non-ionizing blue dye is applied to facilitate the biopsy. The blue-colored draining path away from the tumor is visually distinguished by means of surgery and the physician's direct gaze onto the body tissue.

Photoacoustic imaging is a noninvasive, non-ionizing imaging modality that combines ultrasonic resolution with optical contrast. Photoacoustic imaging relies on the photoacoustic effect to generate pressure waves that can be detected by ultrasound array transducers. Typically, a short laser pulse illuminates tissue leading to optical absorption, followed by rapid heating and thermoelastic expansion to produce pressure waves. Since photoacoustic images can be reconstructed following ultrasound detection with conventional array transducers, it is highly compatible with ultrasound imaging. The same detection mechanism greatly simplifies spatial registration between photoacoustic and ultrasound images.

While photoacoustic imaging is able to image a body based on native tissue contrast, such as from hemoglobin in blood, utilization of contrast agents is able to address a broader spectrum of applications for medical photoacoustic imaging. Methylene blue dye, for example, is routinely used in clinical practice for sentinel lymph node biopsy and is detectable by photoacoustic imaging of the appropriate optical wavelength.

Efficient generation of photoacoustic waves depends on rapid energy deposition that meets the conditions for thermal and stress confinement. In practice, high-energy output, Q-switched pulsed lasers with pulse durations of around 10 ns are used to illuminate tissue for photoacoustic imaging.

For the delivery of high energy laser pulses, a set of mirrors located inside an articulated arm is possible. An articulated arm that incorporates a dye-impregnated polymer rod for optical wavelength conversion is commercially available for cosmetic laser applications (i.e. tattoo removal, etc.) (http://www.conbio.com/cynosure/aesthetic-lasers-sub/revlite) (hereinafter "the Cynosure laser"), but the laser is not designed for imaging. The article: CHULHONG KIM ET AL: "Handheld array-based photoacoustic probe for guiding needle biopsy of sentinel lymph nodes",JOURNAL OF BIOMEDICAL OPTICS, vol. 15, no. 4, 1 January 2010 (2010-01-01), page 046010 discloses an integrated photoacoustic-ultrasound probe delivering laser pulses from a dye laser through a bifurcated optical fiber bundle.

SUMMARY OF THE INVENTION

Aspects of the present invention are directed to addressing one or more of the shortcomings noted above with regard to the prior art.

Contrast agents used in medical photoacoustic imaging are typically sensitive to the optical wavelength. That property is useful in distinguishing the contrast agent signal from the background tissue signals, but it also requires the laser source used for imaging to match the optical absorption spectral characteristics of the contrast agent. Typically, one needs to convert light from a standard laser output wavelength (such as defined by the laser gain medium to be 1064 nanometers (nm) for Nd:YAG, or around 694 nm for ruby, etc.) to the desired wavelength. OPO (optical parametric oscillator) systems and dye lasers are two solutions for doing exactly that.

The ability to perform photoacoustic imaging on a conventional ultrasound scanner requires a compact and rugged laser source that is compatible with the clinical environment. Research lasers currently used for photoacoustic imaging are too bulky, too heavy, and not robust enough to be incorporated into a commercial grade ultrasound scanner or an imaging workstation within a practical footprint.

Ultrasound imaging is a real-time imaging modality, and modern scanners can be moved from bedside to procedure rooms and shared between hospital departments. The development of an ultrasound system with photoacoustics calls for a suitable laser that can accommodate a reasonable degree of portability without the realignment of optical components.

Currently available lasers offer a limited selection of optical wavelengths, which limits the range of optical absorbers (i.e., imaging contrast agents) that can be imaged photoacoustically. Commonly used lasers include Nd:YAG (1064 nm), ruby (694.3 nm), and alexandrite (755 nm) lasers. Harmonic generators can be used to achieve shorter wavelengths, (e.g., harmonic generators with an Nd:YAG laser can reach 532 nm, 355 nm, 266 nm, and 213 nm), at the expense of pulse energy and pulse-to-pulse stability. Functional photoacoustic imaging, or the ability to distinguish optical absorbers based on spectral differences in absorption, requires multiple laser wavelengths.

Generally, tunable lasers are bulky, complex, unreliable (require a sophisticated user maintenance) and expensive, which make them difficult to integrate with conventional ultrasound scanners.

Switching between optical wavelengths would be especially useful for spectroscopic photoacoustic imaging or to differentiate photoacoustic absorbers. However, the high-power tunable lasers available usually need seconds to switch between two wavelengths. According to what is proposed herein below, switching can be done in real time, at 100 Hz for example, thus aiding the ongoing biopsy procedure with real time imaging.

Optical parametric oscillators (OPO) lasers can be pumped for wavelength tuning but they have limited output power. Also, the beam shape and especially the pulse stability are poor.

Dye lasers can also be pumped for wavelength tuning, but the size of commercially available systems is not suitable for compact light delivery for photoacoustic imaging.

The Cynosure cosmetic laser mentioned above is a dye laser, but its pulse repetiton frequency (PRF) of about 2 hertz (Hz) is too low for imaging. Typically, for imaging, a PRF of greater than 20 Hz is desired. Also, the dye-impregnated polymer of the Cynosure cosmetic laser has a relatively low lifetime of only about 10,000 laser shots.

Additionally, powerful medical lasers utilize an articulated arm as a means for light delivery to the point of interest. The articulated arm works well for therapeutic use point by point, but is inadequate for imaging purposes which generally call for high flexibility.

In short, currently available commercial lasers used for photoacoustic imaging suffer from one or more of the following problems. First, the laser size is too big. Second, the wavelength supplied by the laser is not suitable for imaging the desired optical absorber. For instance, the methylene blue absorption peak is 665 nm which is a difficult wavelength to achieve with OPO lasers. Third, the laser is not robust (i.e., reliable) enough or cannot be easily used in a clinical environment. Fourth, combined systems consisting of a pump laser (e.g. Q-switched, Nd:YAG), tunable laser (e.g., OPO or dye laser), and articulated arm for spectroscopic photoacoustic imaging are complex, requiring a complicated alignment, highly skilled user, frequent maintenance, and are too bulky.

What is proposed herein is a compact laser and efficient light delivery system that be used for photoacoustic imaging.

The invention is defined by the claims.

In an aspect of the present disclosure, a photoacoustic medical-imaging device includes a wavelength conversion assembly configured for outputting laser pulses at a targeted wavelength. It further includes a photoacoustic probe configured for acoustic coupling to a patient, for directing said pulses, and for acquiring, in response, radiofrequency data for photoacoustic imaging.

In a sub-aspect, the device further includes an optical fiber bundle.

In a further sub-aspect, the bundle includes an optical fiber having an input end. The device is configured for illuminating, with a homogenous beam, so as to conform to an acceptance angle of the fiber at that end.

In a related sub-aspect, the device includes a light collimator, and a diffuser for receiving the outputted laser pulses from the collimator.

As a further sub-aspect of this, the diffuser is configured for spreading a focus of the pulsed light over an input aperture of an optical fiber bundle to apportion energy of the pulsed light received by different constituent optical fibers of the bundle according to their respective input diameters.

In a yet, further sub-aspect, the device includes a lens configured for receiving pulsed light from the diffuser and for creating the focus to specifically match an acceptance angle.

In a different sub-aspect, the device is configured for outputting other laser pulses at another targeted wavelength. It is further configured for performing image subtraction between image frames acquired with the targeted wavelength and the other targeted wavelength, respectively.

In a further sub-aspect, the targeted wavelength is no more than 690 nanometers and no less than 640 nanometers. The other targeted wavelength is effective for distinguishing an endogenous photoacoustic contrast source.

It is also a sub-aspect that the probe includes at least a portion of an optical fiber bundle.

In a further sub-aspect, the device further comprises an optical coupling system operatively coupled between the bundle and the assembly.

In a yet further sub-aspect, the bundle has an input end that has a light-interface portion, and the system is configured for focusing a homogenous beam of light so as to specifically span the light-interface portion and so as to conform to an acceptance angle.

In one other sub-aspect, at least a portion of the optical fiber bundle is configured based on an optimal size of a beam for photoacoustic imaging. The optimal beam size incident on the patient is a function of both the imaging depth and estimates of respective optical properties of body tissue in the path of the photoacoustic imaging at that depth.

In an additional, structural sub-aspect, the probe features an ultrasound transducer having a lateral direction. The portion of the fiber optic bundle is bifurcated into two branches to deliver the pulses from opposite sides of the transducer. Each of the two branches includes sub-bundles running parallel to the lateral direction.

As a complementary sub-aspect, the probe is configured for at least one of emitting ultrasound for ultrasonic imaging and receiving ultrasound for ultrasonic imaging

In one further sub-aspect, the probe is a handheld probe.

In yet another sub-aspect, the assembly resides in the probe.

In one yet further sub-aspect, the probe is configured for guiding, from the assembly, the pulsed light externally via free space optics and/or a light guide.

As an alternative or complementary sub-aspect, the assembly includes a solid dye-impregnated polymer as a lasing medium.

According to a particular sub-aspect, the assembly includes, for the outputting, a rotational beampath alternator configured for, cyclically at a rate of at least 10 hertz, redirecting: a) to multiple dye cells, a common input beam; and/or b) to a common optical coupling system to a fiber optic bundle, output beams from among respective multiple dye cells.

In one still, different sub-aspect, the device is configured mobile and integrally fixed so as to be portable in a clinical setting from room to room without need for realignment of optical components.

In some versions and as a sub-aspect, the assembly includes a dye for converting to the targeted wavelength.

Likewise, in some versions, the assembly includes a liquid dye as a lasing medium.

As a particular, additional sub-aspect, the probe is configured for dynamically adjusting an optical fiber, advancement- and retraction-wise, responsive to a targeted imaging depth.

Details of the compact photoacoustic medical-imaging device are set forth further below, with the aid of the following drawings, which are not drawn to scale.

## BRIEF DESCRIPTION OF THE DRAWINGS

FIG. 1 is a schematic diagram of a compact laser, and efficient laser pulse delivery, device for photoacoustic imaging;

FIG. 2 is a schematic diagram of a face and side-view of an integrated ultrasound and photoacoustic imaging probe, and side views of other such probes;

FIG. 3 is a flow chart of an integrated ultrasound and photoacoustic imaging probe design process in accordance with the present invention;

FIG. 4 is a schematic diagram of multiple dye cell and real-time wavelength-switching configuration; and

FIG. 5 is a flow chart of interleaving photoacoustic and ultrasonic imaging.

## DETAILED DESCRIPTION OF EMBODIMENTS

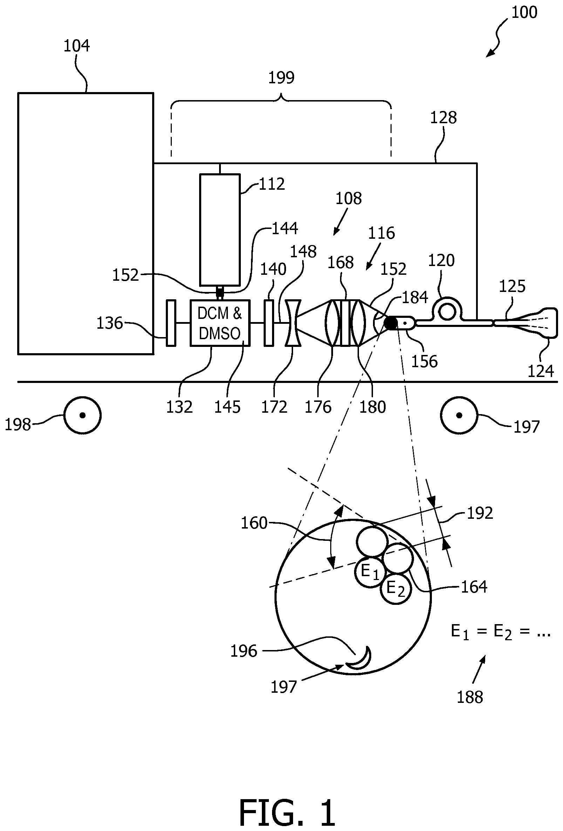

FIG. 1 shows, by illustrative and non-limitative example, a compact laser, and efficient laser pulse delivery, device 100 for photoacoustic imaging. The device 100 includes an ultrasound scanner 104 having a console, a user display such as a screen for presenting two or three-dimensional images, and user controls for imaging. The device further includes a wavelength conversion assembly 108, a pump laser 112, and an optical coupling system 116. A frequency doubled, Nd:YAG laser serves as the pump laser 112. It can be pulsed with a pulse width of less than 200 nanoseconds (ns). One end of an optical fiber bundle 120 joins the optical coupling system 116 to an integrated ultrasound and photoacoustic (hereinafter "US/PA") probe 124 at the other end of the fiber bundle. The probe 124 thus contains a portion 126 of the optical fiber bundle 120. The broken lines in FIG. 1 represent that the portion 126 extends on into the probe 124. A communication line 128 connects the scanner 104 to the pump laser 112 and the probe 124.

The scanner 104 may include a microprocessor or microcontroller having a computer readable medium, such and one or more integrated circuits. The computer readable medium embodies instructions for managing operation of the device 100, which includes controlling ultrasound beamforming, photoacoustic and ultrasonic data acquisition, photoacoustic image subtraction, image display, etc. Operation of the device 100 may be carried out by any combination of software, hardware and firmware.

Photoacoustic images, such as those of a methylene blue dye draining path formed to aid a sentinel lymph node biopsy, are displayable on the user display. Also displayable are ultrasound anatomical imaging, and ultrasound functional imaging such as color flow Doppler imaging.

Components of the wavelength conversion assembly 108 include a dye cell 132, a fully reflective resonator-mirror 136, and a partially reflective resonator-mirror 140. For simplicity of demonstration, the pump laser 112 is pictured as pumping from above the dye cell 132. However, it is actually pumping from the side, so that the pump beam 144 is horizontally aligned with the resonating beams. The wavelength conversion assembly 108 outputs a laser pulse 148 with each pumping pulse 152. As an alternative, the pump laser 112 is placed collinearly. Thus, the fully reflective resonator-mirror 136 would instead be a dichroic mirror, passing the pump beam (e.g., at a wavelength of 532 nm) and fully reflecting emission (e.g., at a wavelength of 645 nm).

The dye cell 132 contains a lasing medium 145, such as 4-dicyanomethylene-2-methyl-6-p-dimethylaminostyryl-4H-pyran (DCM) dye mixed with dimethyl sulfoxide (DMSO). The wavelength conversion is performed by means of organic dyes that fluoresce at a targeted wavelength. The DCM dye mixed with DMSO solvent converts incident 532 nm light into 651 mm light, a suitable wavelength for imaging methylene blue dye accumulation in sentinel lymph nodes. The dye cell 132 further contains a magnetic stirring rod (not shown) to stir the lasing medium 145 to limit dye degradation. The dye cell 132 has a port (not shown) through which the solution can be replaced. Depending on the dye and solvent, they may be replaced after 1 to 24 hours of continuous use.

A feedback mechanism dynamically adjusts laser output on consecutive laser shots to maintain laser stability. The mechanism optically detects degradation of the laser dye or gain medium and heating of resonator components, and may compensate for the degradation by boosting the pump over time.

The wavelength conversion assembly 108 may alternatively be realized as a solid-state dye laser, comprising as a lasing medium 145 a solid dye-impregnated polymer. The entire assembly 108 is replaceable as a unit.

As represented in FIG. 1 by the expanded view of the fiber bundle, the efficient coupling of high-energy laser pulses 148 requires a homogenous laser beam 152 that illuminates an input end 156 of the fiber bundle 120 at all possible incidence angles within the input numerical aperture (or "acceptance angle") 160 of optical fibers 164 comprising the fiber bundle. Since the light 148 from the dye cell 132 is not well collimated, a series of optical components is needed to deliver light from the dye cell into the fiber bundle 120.

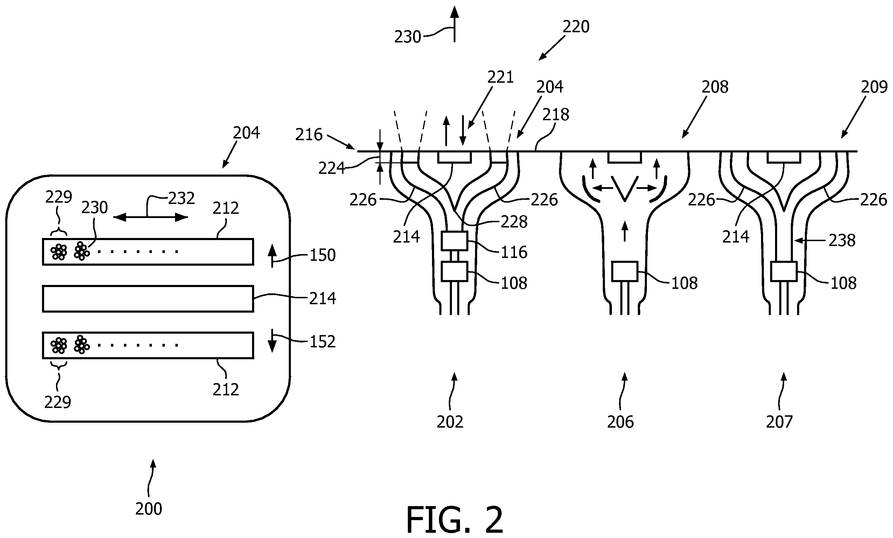

The optical coupling system 116 features a diffuser 168 having a microlens array structure as seen from FIG. 2 of U.S. Patent No. 6,859,326 to Sales. The diffuser 168, also known as an Engineered Diffuser™, can be configured as discussed in the '326 patent, and expanded upon in U.S. Patent No. 6,835,535 to Gretton et al. and U.S. Patent No. 7,033,736 to Morris et al. All three patents are incorporated herein by reference in their entirety. The diffuser 168, in combination with lenses, ensures that a homogenous beam of light is efficiently coupled into the fiber bundle 120. The optical coupling system 116 accordingly further includes a concave lens 172; an input convex lens, or collimator, 176 illuminated by the concave lens; and an output convex lens 180. The two convex lenses 176, 180 sandwich the diffuser 168. The diffuser 168 is configured for spreading a focus of the pulsed light 148 over an input aperture 184 of the optical fiber bundle 120 to apportion energy 188 of the light received by different constituent optical fibers 164 of the bundle 120 according to their respective input diameters 192, i.e., equalize the energy in the usual case of equal diameters. In effect, light of the laser pulse 148 is focused into a small spot which fills the input numerical aperture 160 of the fibers 164, and the diffuser 168 spreads the laser beam focus over the input aperture 184 of the fiber bundle 120 so that each fiber 164 of equal input diameter receives and transmits an equal pulse energy. More particularly, the input end 156 of the fiber bundle 120 has a light-interface portion 196. The light-interface portion 196 is defined herein as a discontinuous end surface that receives light for transmission and thus corresponds to fiber cores and excludes the respective outer claddings 197 that reflectively keep propagating light within the core. The optical coupling system 116 is configured for focusing the homogenous beam 152 so as to specifically span the light-interface portion 196 and so as to conform to an acceptance angle 160 from among those of the constituent optical fibers 164 constituting the fiber bundle 120. The homogenous beam 152 and correct acceptance angle 160 afford efficient light coupling and keep the light incident on the fiber bundle 120 below the fiber damage threshold of constituent fibers 164..

The compact laser, and efficient laser pulse delivery, device 100 for photoacoustic imaging further includes wheels 198 for transportation of the device from room to room in a clinical environment. Relatedly, the optical components 112, 132-140, 168-180 of the wavelength conversion assembly 108 and of the optical coupling system 116 are integrally fixed 199 as a rugged unit for such portability without need for realignment of the optical components. The smaller form factor and fewer components of the device 100 enhance portability.

FIG. 2 presents a face view 200 and a side view 202 of an exemplary, integrated ultrasound and photoacoustic (US/PA) imaging probe 204. It also presents side views 206, 207 of two other, exemplary PA imaging probes 208, 209.

Referring to the face and respective side view 200, 202, the probe 204 has a face 210 that includes two flush, transparent windows 212. The probe 204 includes an ultrasound transducer, or transducer array, 214 that slightly protrudes upward from the face 210. The transducer array 214 is overlaid by an acoustically transmissive covering (not shown). The array 214 is, by means of the covering and a coupling substance, such as an acoustically transmissive gel, configured for being acoustically coupled 216 to the skin 218 of a patient 220. As seen in FIG. 2 from the arrow pair 221, the array 214 can receive ultrasound to acquire radiofrequency (RF) data for photoacoustic imaging, emit ultrasound for ultrasonic imaging, and receive ultrasound bearing RF data for ultrasonic imaging.

Behind each of the two windows 212, by a predetermined, inset distance 224, are the respective output ends of two optical fiber bundle branches 226. The optical fiber bundle 120 is bifurcated 228, within the probe 204, into the two branches 226. The branches 226 slightly bend toward each other in extending to deliver, along an optical path 230, the diffuse laser pulses 148 from opposite sides 150, 152 of the transducer array 214. Each of the two branches 226 is made up of sub-bundles 230 that run parallel to the lateral direction 232. Each sub-bundle 230 is comprised of a subset of the optical fibers 164 in its respective branch 226, and has an aperture size (or "diameter") 229. The sub-bundles 230 each laterally span the entirety of the transducer array 214. Unlike in the embodiment of FIG. 1, both the solid-state wavelength conversion assembly 108 and the optical coupling system 116 are disposed within the probe 204. As part of regular maintenance the assembly 108 can be swapped out. Input to the assembly 108 is carried along an optical fiber bundle, or light pipe, from the pumping laser 112. Unlike in the embodiment of FIG. 1, where only a portion of an optical fiber bundle resides in the probe, the probe 204 of the current embodiment contains an entire optical fiber bundle.

A probe 208 can instead deliver the wavelength-converted light via free-space optics, i.e., by a non-solid transmission medium, such as air. A bifurcating deflecting mirror directs light to curved mirrors on both sides, as shown by the arrows in the side view 206.

The light-guide incorporating probe 209, shown in the side view 207, has a structure that appears similar to the bifurcated optical fiber bundle 120. However, the structure consists of two light guides (or "light pipes") 236 into which a common light guide 238 is bifurcated. Alternatively, a single light guide can be shaped to surround the transducer. Another difference between the two side views 202, 207, is that the light-guide incorporating probe 209 is directly coupled to the wavelength conversion assembly 108.

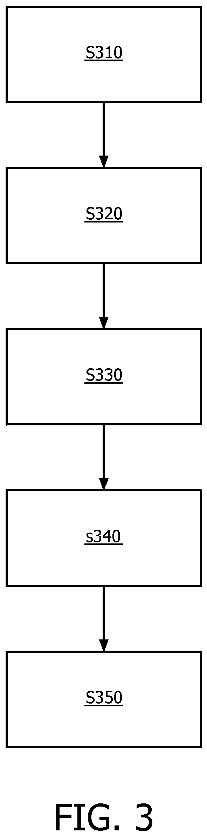

A design process 300 for any of the foregoing US/PA probes 124, 204, 208, according to the present invention, is shown in FIG. 3. The optimal laser beam diameter for delivering optical energy to an embedded target object in biological tissue has been described using Monte Carlo simulation in L. V. Wang, W. R. Chen, and R. E. Nordquist, "Optimal beam size for light delivery to absorption-enhanced tumors buried in biological tissues and effect of multiple beam delivery: a Monte Carlo study," Applied Optics 36, 8286-8291 (1997), and in U.S. Patent No. 6,099,554 to Nordquist et al. Both publications relate to thermal treatment. The methodology can also be applied to imaging. As discussed in the publications, the optimal radius (r) of the incident laser beam , i.e., at its incidence on the skin of a patient, is given by:

$$ \mathrm{r} = [\delta(2\mathrm{d}_t - 2\mathrm{L}_t' + \delta)]^{1/2} $$

where $\delta$ is the optical penetration depth of the tissue, $\mathrm{d}_t$ the distance from the center of the object to the tissue surface, $\mathrm{L}_t'$ is one transport mean free path.

The configuration of the optical fiber bundle 120 (output aperture size 229, position 224 within the probe 124, etc.) can be designed according to this formula to optimize the photoacoustic image for a specific depth, because the laser light emitted by the probe 124 diverges at a predictable rate. The optimal radius r is, as seen in both publications, a function of both the imaging depth $\mathrm{d}_t$ and estimates of respective optical properties of body tissue in a path 230 of said photoacoustic imaging at said imaging depth. The optical properties are the absorption coefficient $\mu_a$, the scattering coefficient $\mu_s$, and the anisotropy g, whose estimations for given body tissue are well known.

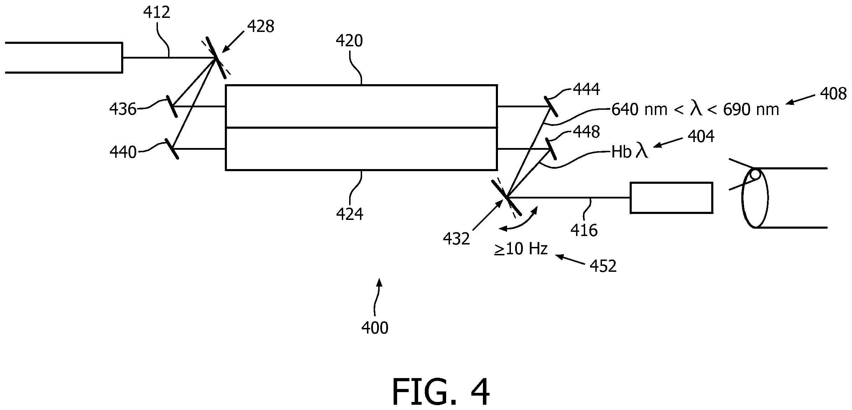

The formula for the optimal radius r is based on the geometry of a single, cross-sectional slice that extends along the central axis of the beam. This is demonstrated in FIG. 4 of the Wang publication. Since the beam may not be round, the actual axial-center-to-periphery distance in the plane of incidence generally varies around the periphery. The optimal radius r is therefore more generally referred to as the "size" of the beam at its incidence on the patient. The beam can be configured such that, for example, its average distance, over the entire periphery, equals its calculated "size r."

For the process 300, an imaging depth $\mathrm{d}_t$ is selected (step S310). An estimate is made of the optical properties (step S320). An estimate is made of the divergence rate, from empirical experience for example (step S330). Based on $\mathrm{d}_t$, $\mu_a$, $\mu_s$ and g, the optimal beam size r is calculated (step S340). The aperture size 229 and the position 224 are calculated based on the optimal beam size r and the divergence rate (step S350). The first two steps (steps S310, S320) can be performed in either order. The divergence rate estimate (step S330) can be done any time prior to the bundle physical characteristics calculation step (S350).

The probe 124 may also be designed to dynamically adapt to the targeted imaging depth $\mathrm{d}_t$ defined by a current ultrasound setting. The setting might be a receive-beamforming input parameter, such as imaging depth or another operator-furnished parameter. Thus, the bundle branches 226 are advanced or retracted, thereby changing the inset distance 224, to optimize photoacoustic imaging at that depth. Fixed annular outer sleeves for the branches 226 are both pushed or pulled, correspondingly for advancement or retraction, by a probe-resident motorized mechanism that is responsive to the operator-furnished receive-beamforming input parameter. Because the branches 226, like the fibers 164, are flexible, slack within the probe 124 allows for the advancement and retraction. Therefore, the probe 124 is configured for dynamically adjusting an optical fiber 164, advancement- and retraction-wise, responsive to the targeted imaging depth. A further possibility is that the advancement/retraction instead rely on manual adjustment, in parallel for example, as by a graduated thumbwheel on the probe 124.

FIG. 4 depicts one possible multiple dye cell and real-time wavelength-switching configuration 400 serving as a wavelength conversion assembly. For enhancement, a photoacoustic image acquired at one targeted, illumination wavelength 404 is subtracted from a photoacoustic image acquired at another targeted, illumination wavelength 408. This is similar to ultrasound pulse inversion imaging. The first targeted wavelength 404 is selected to be close to the methylene blue optical absorption peak of 665 nm, but may be greater than 640 nm and less than 690 nm. The second targeted wavelength 408 is selected so as to be effective for distinguishing an endogenous photoacoustic contrast source. An example would be hemoglobin (Hb), optically distinguishable from methylene blue with a wavelength between 700 and 900 nm, or melanin. Any endogenous source of photoacoustic contrast, in combination with any internally-introduced exogenous photoacoustic contrast agent, is within the intended scope of what is proposed herein. Likewise, targeted uptake or bearing of different photoacoustic agents by respective adjacent tissues may be distinguished.

The switching configuration 400, in response to being pumped by an input laser beam 412 from the pump laser 112, outputs a laser beam 416 of the targeted wavelength to the optical coupling system 116. There, as discussed herein above, the beam 416 is conformed to the acceptance angle 160 of the constituent optical fibers 164 constituting the fiber bundle 120.

The configuration 400 includes multiple dye cells. Two dye cells 420, 424 are shown in FIG. 4. The configuration 400 further includes optical switching and alignment optics. The optics includes two rotational beam-path alternators 428, 432, and four fixed deflecting mirrors 436, 440, 444, 448. Each alternator 428, 432 has two light-deflecting orientations, one shown in FIG. 4 as a solid line and the other shown as a broken line. The alternator 428, 432 may be implemented as a fast scanning mirror galvanometer used to switch the respective beam 412, 416 on consecutive laser shots to achieve the alternating laser wavelengths 404, 408. Other mechanisms can be used for beam deflection between the two dye cells 420, 424, such as a rotating prism as in a compact, liquid crystal on silicone (LCoS) engine. Switching between the two orientations occurs cyclically at a rate 452 of at least 10 hertz. It may occur every 10 milliseconds (ms), for example. The optics may be implemented instead only at the input or only at the output. Accordingly, the configuration 400 redirects at least one of: a) to the multiple dye cells 420, 424, a common input beam 412; and b) to a common optical coupling system 116 to the fiber optic bundle 120, output beams 416 from among respective ones of the multiple dye cells.

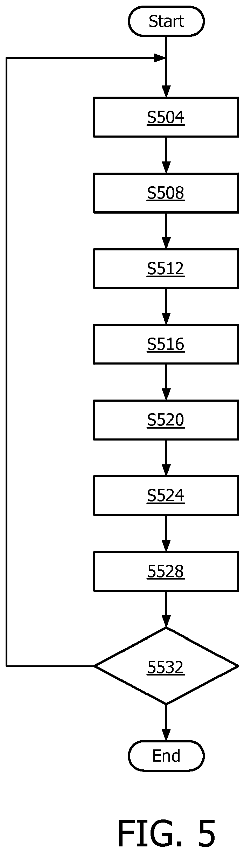

Photoacoustic and ultrasonic imaging are interleaved for the compact laser, and efficient laser pulse delivery, device 100 as seen from FIG. 5. A pulse is fired from the first dye cell 420 (step S504). From the echoed back RF data a photoacoustic image is acquired (step S508). A pulse is fired from the second dye call 424 (step S512). From the echoed back RF data a photoacoustic image is acquired (step S516). By subtraction of one image from the other, a difference image is formed (step S520). Ultrasound is emitted and received to acquire a frame of ultrasound data (step S524). The ultrasound image and difference image are displayed (step S528). If a next image frame is to be displayed (step S532), the process repeats from the beginning at step S504. The subtraction step S520 and the ultrasound acquisition step S524 can be performed in either order. For the display step S528, the PA image may be superimposed on a grayscale B-mode ultrasound image. Or they may be displayed side-by-side. The combined B-mode/PA image may be displayed side-by-side with a Doppler image, such as a color flow image, or the color flow image can color code the combined image as an additional overlay.

A photoacoustic medical-imaging device includes a wavelength conversion assembly configured for outputting laser pulses at a targeted wavelength. It also includes a photoacoustic probe configured for acoustic coupling to a patient, for directing the pulses, and for acquiring, in response, radiofrequency data for photoacoustic imaging. It may include an optical fiber bundle that comprises an optical fiber having an input end, and be configured for illuminating, with a homogenous beam, so as to conform to an acceptance angle of the fiber at that end. It may also include a light collimator, and a diffuser for receiving the outputted laser pulses from the collimator. The diffuser may be configured for spreading a focus of the pulsed light over an input aperture of the bundle to equalize the light received by different constituent optical fibers of the bundle. The assembly may include a dye cell, and may reside in the device.

The photoacoustic medical-imaging device 100 has a broad range of applications related to optical imaging, especially photoacoustic imaging. Of particular interest is image-guided sentinel lymph node biopsy. It also has applications for cancer diagnosis/staging, imaging of angiogenesis, functional imaging (i.e. oxygen saturation and/or total concentration of hemoglobin), and treatment monitoring. The compact light delivery system also has application for photoacoustic molecular imaging, particularly for distinguishing contrast agents from background signals using spectroscopic photoacoustic imaging.

While the invention has been illustrated and described in detail in the drawings and foregoing description, such illustration and description are to be considered illustrative or exemplary and not restrictive; the invention is limited by the claims. Outside the scope of the claimed invention, the optimal radius calculation, mentioned above in the context of the optical fiber sub-bundle 230, may instead be applied for configuring a probe-resident light pipe as to its radius and distance from the transducer/patient interface. Also, although pulse-echo ultrasound by a single probe is demonstrated, a setup requiring a second, remote ultrasound receiver of the probe-emitted ultrasound, or in which the probe receives ultrasound that is remotely emitted, may be obtained outside the scope of the claimed invention.

A computer program can be stored momentarily, temporarily or for a longer period of time on a suitable computer-readable medium, such as an optical storage medium or a solid-state medium. Such a medium is non-transitory only in the sense of not being a transitory, propagating signal, but includes other forms of computer-readable media such as register memory, processor cache, RAM and other volatile memory.

A single processor or other unit may fulfill the functions of several items recited in the claims.

**Claims**

1. A method for configuring an integrated ultrasound and photoacoustic imaging probe (124, 204), including providing at least a portion of an optical fiber bundle (120) within said probe,

the at least portion being bifurcated into two optical fiber bundle branches (226) having a light output end, and being provided positioned within the probe with said light output end at an inset distance (224) from an output face (210) of the probe, and

each optical fiber bundle branch (226) comprising sub-bundles (230) having ends located at said light output end, each defining an output aperture having a size (229), said method being **characterized by** comprising the steps of:

> selecting an imaging depth (S310);
> estimating optical properties of a body tissue in a path (230) of said photoacoustic imaging (S320);
> estimating a divergence rate of a laser beam (S330), said laser beam to be utilized in photoacoustic imaging optimized for said imaging depth;
> calculating an optimal incident beam size (S340) on the skin of a patient based on the optical properties of body tissue and said imaging depth;
> computing (S350), based on the calculated optimal beam size and the estimated divergence rate, the sub-bundle aperture size (229) and the inset distance (224) within said probe; and
> configuring said at least a portion within the probe (124, 204) based on the computed aperture size (229) and the computed inset distance (224).

2. The method of claim 1, said beam size (229) being a radius of said beam.

3. The method of claim 1, further comprising providing a wavelength conversion assembly configured for outputting laser pulses at a targeted wavelength (404);
   wherein the probe is configured for acoustic coupling (216) to a patient, for directing said pulses, and for acquiring, in response, radiofrequency data for photoacoustic imaging; and
   the method further comprising providing an ultrasound transducer (214) for the probe having a lateral direction (232), wherein said provided portion is bifurcated into two branches for delivering in use said pulses from opposite sides of said transducer, each of the two branches comprising sub-bundles running parallel to said direction.

4. The method of claim 3, wherein said inset distance is a distance from said output face of the probe to a light-emitting end of a sub-bundle from among said sub-bundles (230).

5. The method of claim 1, said at least a portion being merely a portion.

6. The method of claim 1, said at least a portion including the entire optical fiber bundle (120).

7. The method of claim 1, further comprising providing a wavelength conversion assembly configured for outputting laser pulses at a targeted wavelength (404);
   wherein the probe is configured for acoustic coupling (216) to a patient, for directing said pulses, and for acquiring, in response, radiofrequency data for photoacoustic imaging; and
   the method further comprising providing a scanner (104) configured for deriving said photoacoustic imaging from said radiofrequency data.

**Patentansprüche**

1. Verfahren zum Konfigurieren einer integrierten Ultraschall- und fotoakustischen Bildgebungssonde (124, 204),
   beinhaltend das Bereitstellen mindestens eines Abschnitts eines Lichtwellenleiterbündels (120) innerhalb der Sonde,
   wobei der mindestens Abschnitt in zwei Lichtwellenleiterbündelverzweigungen (226), aufweisend ein Lichtabgabeende, gegabelt sein kann und innerhalb der Sonde mit dem Lichtabgabeende in einem Einsetzabstand (224) von einer Abgabefläche (210) der Sonde positioniert bereitgestellt ist und
   jede Lichtwellenleiterbündelverzweigung (226) Unterbündel (230) umfasst, die an dem Lichtabgabeende befindliche Enden aufweisen, jeweils eine Abgabeöffnung definierend, die eine Größe (229) aufweist;
   wobei das Verfahren **dadurch gekennzeichnet ist, dass** es die folgenden Schritte umfasst:

> Auswählen einer Bildgebungstiefe (S310);
> Schätzen optischer Eigenschaften eines Körpergewebes in einem Pfad (230) der fotoakustischen Bildgebung (S320);
> Schätzen einer Divergenzrate eines Laserstrahls (S330), wobei der Laserstrahl bei für die Bildgebungstiefe optimierter fotoakustischer Bildgebung angewendet wird,
> Berechnen einer optimalen Größe des auf die Haut eines Patienten einfallenden Strahls (S340) basierend auf den optischen Eigenschaften von Körpergewebe und der Bildgebungstiefe;
> Berechnen (S350), basierend auf der berechneten optimalen Strahlgröße und der geschätzten Divergenzrate, der Unterbündelöffnungsgröße (229) und des Einsetzabstands (224) innerhalb der Sonde; und
> Konfigurieren des mindestens Abschnitts innerhalb der Sonde (124, 204) basierend auf der berechneten Öffnungsgröße (229) und dem berechneten Einsetzabstand (224).

2. Verfahren nach Anspruch 1, wobei die Strahlgröße (229) ein Radius des Strahls ist.

3. Verfahren nach Anspruch 1, weiter umfassend das Bereitstellen einer Wellenlängenumwandlungsanordnung, die zum Abgeben von Laserimpulsen bei einer gezielten Wellenlänge (404) konfiguriert ist; wobei die Sonde zum akustischen Koppeln (216) an einen Patienten, zum Lenken der Impulse und zum Erfassen, in Reaktion darauf, von Hochfrequenzdaten zur fotoakustischen Bildgebung konfiguriert ist; und
das Verfahren weiter das Bereitstellen eines Ultraschallwandlers (214) umfasst, damit die Sonde eine laterale Richtung (232) aufweist, wobei der bereitgestellte Abschnitt zum Übermitteln, bei Verwendung, der Impulse von entgegengesetzten Enden des Wandlers in zwei Verzweigungen gegabelt ist, wobei jede der zwei Verzweigungen parallel zu der Richtung verlaufende Unterbündel umfasst.

4. Verfahren nach Anspruch 3, wobei der Einsetzabstand ein Abstand von der Ausgangsfläche der Sonde zu einem lichtemittierenden Ende eines Unterbündels zwischen den Unterbündeln (230) ist.

5. Verfahren nach Anspruch 1, wobei der mindestens Abschnitt lediglich ein Abschnitt ist.

6. Verfahren nach Anspruch 1, wobei der mindestens Abschnitt das gesamte Lichtwellenleiterbündel (120) beinhaltet.

7. Verfahren nach Anspruch 1,
weiter umfassend das Bereitstellen einer Wellenlängenumwandlungsanordnung, die zum Abgeben von Laserimpulsen bei einer gezielten Wellenlänge (404) konfiguriert ist;
wobei die Sonde zum akustischen Koppeln (216) an einen Patienten, zum Lenken der Impulse und zum Erfassen, in Reaktion darauf, von Hochfrequenzdaten zur fotoakustischen Bildgebung konfiguriert ist; und
das Verfahren weiter das Bereitstellen eines Scanners (104) umfasst, der zum Ableiten der fotoakustischen Bildgebung von den Hochfrequenzdaten konfiguriert ist.

**Revendications**

1. Procédé de configuration d'une sonde (124, 204) d'imagerie ultrasonore et photoacoustique intégrée, incluant la fourniture d'au moins une partie d'un faisceau (120) de fibres optiques à l'intérieur de ladite sonde,
l'au moins une partie étant divisée en deux branches (226) de faisceau de fibres optiques ayant une extrémité de sortie de lumière, et étant fournie positionnée à l'intérieur de la sonde avec ladite extrémité de sortie de lumière à une distance d'insertion (224) d'une face de sortie (210) de la sonde, et
chaque branche (226) de faisceau de fibres optiques comprenant des sous-faisceaux (230) ayant des extrémités situées au niveau de ladite extrémité de sortie de lumière, chacune définissant une ouverture de sortie ayant une taille (229),
ledit procédé étant **caractérisé par le fait qu'**il comprend les étapes de :

sélection d'une profondeur d'imagerie (S310) ;
estimation de propriétés optiques d'un tissu corporel dans un chemin (230) de ladite imagerie photoacoustique (S320) ;
estimation d'un taux de divergence d'un faisceau laser (S330),
ledit faisceau laser devant être utilisé dans une imagerie photoacoustique optimisée pour ladite profondeur d'imagerie ;
calcul d'une taille optimale de faisceau incident (S340) sur la peau d'un patient sur la base des propriétés optiques du tissu corporel et de ladite profondeur d'imagerie ;
calcul (S350), sur la base de la taille optimale calculée du faisceau et du taux de divergence estimé, de la taille (229) d'ouverture du sous-faisceau et de la distance d'insertion (224) à l'intérieur de ladite sonde ; et
configuration de ladite au moins une partie à l'intérieur de la sonde (124, 204) sur la base de la taille (229) d'ouverture calculée et de la distance d'insertion (224) calculée.

2. Procédé selon la revendication 1, ladite taille (229) de faisceau étant un rayon dudit faisceau.

3. Procédé selon la revendication 1, comprenant en outre la fourniture d'un ensemble conversion de longueur d'onde configuré pour délivrer en sortie des impulsions laser à une longueur d'onde (404) ciblée ;
dans lequel la sonde est configurée pour un couplage acoustique (216) à un patient, pour diriger lesdites impulsions, et pour acquérir, en réponse, des données de radiofréquence pour imagerie photoacoustique ; et
le procédé comprenant en outre la fourniture d'un transducteur ultrasonore (214) pour la sonde ayant une direction latérale (232), dans lequel ladite partie fournie est divisée en deux branches pour délivrer en cours d'utilisation lesdites impulsions à partir de côtés opposés dudit transducteur, chacune des deux branches comprenant des sous-faisceaux orientés parallèlement à ladite direction.

**4.** Procédé selon la revendication 3, dans lequel ladite distance d'insertion est une distance de ladite face de sortie de la sonde à une extrémité électroluminescente d'un sous-faisceau parmi lesdits sous-faisceaux (230).

**5.** Procédé selon la revendication 1, ladite au moins une partie étant simplement une partie.

**6.** Procédé selon la revendication 1, ladite au moins une partie incluant le faisceau (120) entier de fibres optiques.

**7.** Procédé selon la revendication 1,
comprenant en outre la fourniture d'un ensemble conversion de longueur d'onde configuré pour délivrer en sortie des impulsions laser à une longueur d'onde (404) ciblée ;
dans lequel la sonde est configurée pour un couplage acoustique (216) à un patient, pour diriger lesdites impulsions, et pour acquérir, en réponse, des données de radiofréquence pour imagerie photoacoustique ; et
le procédé comprenant en outre la fourniture d'un scanner (104) configuré pour dériver ladite imagerie photoacoustique à partir desdites données de radiofréquence.

FIG. 1

FIG. 2

EP 2 884 886 B1

S310

S320

S330

s340

S350

FIG. 3

FIG. 4

FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6859326 B, Sales
- US 6835535 B, Gretton

- US 7033736 B, Morris
- US 6099554 A, Nordquist

**Non-patent literature cited in the description**

- **CHULHONG KIM et al.** Handheld array-based photoacoustic probe for guiding needle biopsy of sentinel lymph nodes. *JOURNAL OF BIOMEDICAL OPTICS,* 01 January 2010, vol. 15 (4), 046010

- **L. V. WANG ; W. R. CHEN ; R. E. NORDQUIST.** Optimal beam size for light delivery to absorption-enhanced tumors buried in biological tissues and effect of multiple beam delivery: a Monte Carlo study. *Applied Optics,* 1997, vol. 36, 8286-8291